# EUROPEAN PATENT APPLICATION

(11) **EP 1 717 321 A1**
(43) Date of publication of application: **02.11.2006**
(21) Application number: 05425272.1
(22) Date of filing: 29.04.2005
(51) Int. Cl.: C12Q 1/68

(54) **System for surface identification of commercial products using DNA tracer**

(71) Applicant: Avvocato Alberto Franchi, 37122 Verona (IT)
(72) Inventor: Franchi, Alberto, 37025 Verona (IT)
(74) Representative: Modiano, Micaela Nadia

(57) **Abstract**

A DNA tracer and a system for surface identification of commercial products using the tracer are herein described, wherein the tracer comprises at least one DNA cloning vector type in which one or more DNA fragments are inserted which are mutually different at least in terms of length or sequence, wherein the inserted DNA is primary DNA, each inserted fragment has a length of at least 50 base pairs (hereinafter, b.p.), and the inserts are at least two if the inserted DNA is derived entirely from the product to be identified.

## Description

The present invention relates to a surface identification system for commercial products, particularly foodstuffs, even more particularly horticultural products, which uses a DNA-based tracer and allows univocal identification of characteristics of commercial products on the surface of which the tracer is applied, in particular of the traceability of said product.

Recent and severe accidents in the agri-foodstuff sector (for example the case of the presence of dioxin in pork meat) have highlighted the importance for public health of traceability (also known as trackability) of foodstuffs within the production chain.

January 1st, 2005, after approximately three years of *vacatio legis,* the first part of the operational obligations imposed by Community Regulation 178/2002 EC came into force in Italy; "traceability" stands out among these obligations. Traceability, according to regulation 178/02 EC, is an intercompany process that allows to identify the individual components of the production cycle both upstream and downstream, by means of the information that each business operator is required to store, and exhibit on demand, regarding the level that precedes and follows his, in the process for forming a foodstuff.

Specifically, article 18 of regulation EC 178/2002 states:
*"Traceability*
   *1. The traceability of food, feed, food-producing animals, and any other substance intended to be, or expected to be, incorporated into a food or feed shall be established at all stages of production, processing and distribution.*
   *2. Food and feed business operators shall be able to identify any person from whom they have been supplied with a food, a feed, a food-producing animal, or any substance intended to be, or expected to be, incorporated into a food or feed. To this end, such operators shall have in place systems and procedures which allow for this information to be made available to the competent authorities on demand.*
   *3. Food and feed business operators shall have in place systems and procedures to identify the businesses to which their products have been supplied. This information shall be made available to the competent authorities on demand.*
   *4. Food or feed which is placed on the market or is likely to be placed on the market in the Community shall be adequately labelled or identified to facilitate its traceability, through relevant documentation or information in accordance with the relevant requirements of more specific provisions. [...]"*

In the context of the production and marketing of meats, for example, the traceability that is prescribed is so precise and demanding as to allow to go back from the final package of the product to the animal, to its history from birth and to every passage from the farm to the slaughter to subsequent cutting and marketing.

Although the current and most widespread interpretation of the concept of traceability applies to the assurance of safety of the foodstuff, and therefore as a compulsory requirement of all foodstuffs, the same expression of traceability is currently used also with reference to very different contents and purposes. It is in fact also possible to understand the concept of traceability as a tool for ensuring global quality and specificity of any food or non-food product. From this viewpoint, therefore, traceability becomes a voluntary tool for adding value to said products. The present invention belongs to a broader concept of production chain traceability, such as the traceability cited for example for the first time in the guidelines of the *"Polo per la Qualificazione Agro-industriale"* ("Center for Agroindustrial Qualification") of the Faculty of Agriculture of the University of Milan, according to which *"production chain traceability is the documented identification of all the companies that have contributed to the production and marketing of units of product that can be identified physically and individually".* This definition identifies a new model, known as *Responsabilità della Filiera di Prodotto* (Product Chain Responsability), abbreviated hereinafter as *RFP.*

Ordinary systems for surface identification and labeling of commercial products, particularly systems suitable to provide traceability of food products, generally are based on ordinary labels, optical identification systems (bar codes), radiofrequency systems, and systems that use the genetic code of the product to be labeled. However, these last have been applied so far only to the meat trade and exclusively for the purpose of product traceability in the classic sense. The systems mentioned above, therefore, do not provide a valid solution if one wishes to have information regarding the labeled product other than mere traceability. Moreover, the relatively limited amount of information that can be applied to conventional labels and the relative ease with which they can be forged, like bar codes, in principle cause normal systems for surface identification of products to be unsuitable to meet the requirements for example of the RFP model mentioned above. Moreover, conventional systems for surface identification of products, especially in the case of food products, do not allow to trace the status and conditions of preservation and processing of said products by means of a simple analysis of the labels and of the identification elements of the system considered.

The aim of the present invention is therefore to provide a system for surface identification of commercial products that overcomes the drawbacks of conventional systems.

Within this aim, an object is to provide a surface identification system for commercial products that allows to identify simultaneously and uniquely, quickly and at low cost several items of information related to a commercial product.

Another object is to provide an identification system that uses a new identification element (hereinafter termed tracer), which can be applied on a surface easily in order to identify uniquely various commercial products that belong even to very different categories.

Another object is to provide an identification system and the corresponding tracer that allow traceability of a horticultural product within the food production chain.

Another object is to provide a tracer that is suitable for use in a system as defined above and allows to identify one or more aspects of a group of commercial products, without relying, if so required, on the genetic characteristics of the individual products that belong to the group to be identified.

This aim and these and other objects are achieved by a DNA tracer, particularly for surface identification of a commercial product on which it is applied, said tracer comprising at least one type of DNA cloning vector, in which one or more fragments of DNA that are mutually different at least in terms of length or sequence are inserted, wherein:
- the inserted DNA is primary DNA,
- each inserted fragment has a length at least equal to 50 base pairs (hereinafter b.p.),
- if the inserted DNA derives entirely from the product to be identified, the inserts are at least two.

The aim and objects of the invention are also achieved by a method for identifying a commercial product, which comprises the steps of:
i) providing one or more mutually different DNA tracers as defined above, and
ii) storing said tracers in a database, assigning uniquely to each tracer a "user code" entry.

The aim and objects of the invention are also achieved by the use of a DNA tracer as defined above for the surface identification of a commercial product on which the tracer is applied, said product been preferably a food product, more preferably a horticultural product.

It is understood that the characteristics described with reference to an individual aspect of the invention are to be considered valid, despite not being repeated explicitly, also as regards other aspects if they are applicable thereto.

In a first aspect, the invention relates to a DNA tracer as defined above.

Identifying a commercial product comprises detecting at least one characteristic thereof by analyzing the DNA tracer applied thereto. If the detected characteristics are two or more, their detection occurs simultaneously, since it requires a single analysis of each DNA fragment inserted in the vector. In a preferred embodiment, the detected characteristics are one or more of the following:
- general traceability of the product in the production chain,
- geographical production area,
- producer or vendor,
- production batch,
- characteristics of the processing to which the product has been subjected before its final use,
- state of preservation, advantageously if the product is of the food type,
- contamination with GMO (genetically modified organisms).

In one embodiment, the detected characteristic is at least one among: general traceability of the product in the production chain, geographical production area, producer or vendor; preferably, the characteristics that are detected are all the ones cited above.

In a preferred embodiment, the characteristics of the product that are obtained by analyzing the tracer are relevant in terms of the traceability of said product. In particular, if the analyzed product is a food product, preferably a horticultural product, the invention in all of its aspects allows traceability of the product in the broadest meaning of this term as defined in relation to the RFP model.

The invention therefore is, inter alia, an alternative to the classic informational labelling applied to the surface of products, thus allowing to obtain, with a single analysis of the tracer, a plurality of pieces of information simultaneously, for example the information needed to identify with assurance the geographical origin of the product or its producer or also its traceability within the production chain.

Moreover, since the core of the DNA tracer is constituted by organic material that is potentially degradable and further can be removed from the surface of the product to which it is applied, the analysis of said tracer can be useful in order to obtain information regarding the state of preservation of the product, regarding any bacterial contamination in progress or undergone by the product during its industrial history, and regarding the processing and preservation conditions to which the product has been subjected (such as for example treatments with UV rays, heat treatments, or washing). This information is in fact directly evident to the eyes of any person skilled in the art as a consequence for example of difficulty in analyzing the tracer, obtaining quantities of tracer different from the ones initially applied to the product (therefore due to partial removal of the tracer) or identifying a tracer that is partially or completely degraded or modified.

The expression "DNA cloning vector" (also just "vector" hereinafter) is used to designate any DNA molecule that is known to replicate itself autonomously in a host cell, in which it is possible to insert one or more foreign segments of DNA for example by means of splicing techniques, so as to allow replication of the inserted foreign DNA. Examples of vectors are plasmids, bacteriophages and cosmids. A highly preferred vector is a plasmid, i.e., a circular DNA molecule that is capable of replicating itself separately with respect to the genetic makeup of the host cell in which it is inserted. The plasmids that can be used are any of the plasmids that are commercially available.

The expression "vector type" is used to designate the combination of vector and pattern of DNA inserts. Two vector types differ necessarily in at least one of the following aspects: length and sequence of the DNA inserts. Preferably, vector types differ in both of these aspects and optionally also in terms of the number of inserts. If there are two or more vector types in a tracer, the characteristic of each tracer is constituted by the pattern of all the DNA inserts that are present. Due to the fact that two vector types can never be identical (as their respective inserts differ at least in terms of length or sequence), the insert pattern is unique for each vector type. Thanks to the use of different vector types, it is therefore possible to create a practically infinite pool of mutually different tracers, which are useful for uniquely identifying a specific product or a set of products by means of one or more "product codes" constituted by said tracers.

The tracer according to the invention must have at least one vector type with one or more DNA inserts, preferably two or more types. If all the DNA inserted in a vector derives from the genoma of the product to be identified, the inserts are at least two. The term "derive" from the product or producer is understood to mean that the inserted DNA fragment or fragments correspond entirely to one or more portions taken from the genetic code of the product to be identified or of the producer/vendor (therefore excluding the case in which the one or more fragments are constituted entirely or partially by synthetic DNA) without further modifications affecting the sequence and length of the fragment or fragments.

The length of the shortest fragment to be inserted must be at least 50 b.p. This minimum length is not an absolute technical limit for DNA analysis, but rather is the value that has been found to correspond to the lower limit for being able to perform analysis of the DNA fragments inserted in the tracer in a short time, at low cost and so as to obtain precise and accurate results by using normal recombinant DNA techniques. In other words, one of the advantages of the present invention is that it allows to identify several characteristics of a specific product in a short time and with a relatively low financial effort by means of a single analysis of the tracer. Therefore, although modem recombinant DNA technologies would allow the analysis of the number, length and specific sequence of fragments even shorter than 50 b.p., it has been found that the analysis of these fragments might cause the overall identification process to be excessively long and economically less attractive and to yield results that are potentially less precise and accurate.

Preferably, the DNA fragments inserted in the vector have a length comprised between 50 b.p. and 1000 b.p., more preferably comprised between 100 b.p. and 1000 b.p.

In one embodiment of the invention, the tracer comprises a single vector type. In this embodiment, the variables that can be analyzed and by means of which the product to which the tracer has been applied is identified are therefore the sequence of bases of the individual inserted DNA fragments, the number of inserted DNA fragments, and their length.

In a highly preferred different embodiment, the tracer comprises more than one vector type. In this embodiment, the possible combinations of variables for identifying the analyzed product are considerably increased, since the number of possible combinations of different fragments is higher.

The insertion of the DNA fragments in the vectors and the techniques for analyzing them in order to extract information from the specific tracer are performed in accordance with conventional techniques that are known as a whole to any person skilled in the art as "recombinant DNA technology". In particular, the production of the vectors modified with DNA insertion occurs for example by means of conventional techniques for splicing, host cell selection, vector quantity amplification and resulting vector purification. Likewise, analysis of the tracer in order to extract the pattern of the DNA inserts can be performed with conventional techniques based for example on PCR, advanced PCR or real-time PCR. In a particular embodiment, the tracer is recovered from the product to which it had been applied by taking a sample of the surface of said product and/or by removing by abrasion the tracer from the surface of the product by using instruments and surfaces that are commonly known in the field of genetic engineering as being suitable for the purpose, or also, as described in detail hereinafter, by removing the sticker the adhesive whereof contains the vector with the corresponding DNA inserts. The person skilled in the art in any case will know how to apply other conventional analysis methods according to the specific requirements of the situation, by relying on ordinary knowledge of the sector and/or with routine experiments.

The expression "primary DNA" is used to specify that the DNA inserts in the vector are constituted by DNA with primary structure.

The DNA that can be used in the context of the present invention is selected among natural DNA, synthetic DNA (i.e., DNA synthesized with conventional chemical technologies), or a combination of both. The natural DNA can be derived from animals, plants or a combination of both.

The DNA that can be used in the invention is to be understood as a sequence of the natural or chemically modified deoxyribonucleotide bases without therefore necessarily being DNA that codes for a protein or parts thereof.

In one embodiment, each DNA fragment of a vector is extracted directly from the gene code of the product to be identified (i.e., it is derived therefrom), on condition that said product contains DNA.

In a different and preferred embodiment, each DNA fragment of a vector derives from the genoma of the producer or of one of the producers or of one or more of the vendors of the specific product that is to be identified. In this embodiment, it is possible to identify, by using the same tracer or a class of tracers that are different but characterized by considerable similitude, an entire class of products, in which said products need not contain per se genetic material.

In a different and preferred embodiment, it is possible to assemble in the laboratory each DNA fragment of a vector, on condition of not producing two fragments that are identical in terms of sequence and length.

In another embodiment, the DNA inserts in each vector type are more than one and are a combination of at least two of the following possibilities: DNA derived from the product, DNA derived from the producer/vendor, and totally or partially synthetic DNA.

The use of DNA that is not derived entirely from the product to be marked (but is derived for example from the genetic makeup of the producer or from synthetic DNA) is a particularly preferred solution, especially when one wishes to mark horticultural products, or a necessary solution when the commercial product does not contain DNA of its own. Among the many advantages that can be obtained by not using the DNA of the product to be marked, mention can be made for example of the elimination of the risk of not distinguishing the different origin of two samples of meat or vegetables that derive respectively from twin animals or from the same plant variety, or the possibility to mark with a single tracer or with a family of tracers with a high degree of similarity different categories of products that however share one or more factors, such as origin from a same geographical area or from a single producer. These advantages overcome the technical obstacles that so far have made it unthinkable to apply DNA traceability for horticultural products, since the uniqueness of the genetic makeup of an animal cannot be found, and consequently utilized, for example for a vegetable or fruit.

Optionally, the tracer according to the invention also comprises at least one application agent, which is suitable to optimize the applicability of said vector to the surface of the product that is to be identified.

The expression "agent suitable to make the vector applicable" (application agent) is used to designate any substance or mixture of substances commonly applied to the surface of commercial products for example for preservation or protection of said product and acting as a matrix inside which the tracer vectors are added. Preferably, the agent is any substance or mixture of substances used in the food sector, more preferably in the agri-foodstuff sector. Advantageously, the agent is therefore a substance or mixture of substances that are edible and non-toxic or in any case whose use in the food sector is accepted by currently applicable statutory provisions. The use of an edible agent allows to mark the food product or a class of food products with a label that is advantageously edible and whose chemical nature allows to highlight bacterial contaminations or particular industrial manipulation treatments.

As mentioned, the tracer must be applied on the surface. It can be applied to all or part of the surface of the product to be marked according to various methods, matched by different application agents. For example, it is possible to apply the tracer according to one or more of the methods selected among: full or partial immersion of the product, atomization of the entire or partial surface of the product, spreading of the entire or partial surface of the product, or any other method commonly used for example in the food sector and immediately evident to the person skilled in the art.

In one embodiment, the tracer is applied to the surface of the product in the pure state, i.e., without the application agent. Although it is particularly expensive, this embodiment can meet the need of certain fields in which one does not wish, or is required to avoid, contamination of the product to be marked with other foreign elements apart from the vectors of the tracer.

In a different embodiment, the vectors are applied after mixing in an application agent as defined above.

In one embodiment, the application agent is selected among waxes and resins of vegetable and/or chemical origin and mixtures thereof that are normally used in the food sector. Preferred agents are one or more among Carnauba wax, caoutchouc, beeswax, xanthan gum.

In a different embodiment, the application agent is selected among liquid and/or semiliquid substances of vegetable, animal, mineral and/or chemical origin normally used in the food sector. Preferred agents are one or more adhesives of the food-derived type (for example fish glue), food-derived lacquers (for example sealing wax) and food-derived inks. The mentioned categories comprise natural and synthetic substances. These substances are commonly used, easily commercially available and immediately identifiable by a person skilled in the art (particularly in the food sector) on the basis of normal knowledge of the field.

In a different embodiment, the application agent is selected among flours and powders that are advantageously edible, preferably vegetable flours (for example corn flour), animal flours, chemical flours, mineral flours (for example silica powder), and mixtures thereof. Preferably, said flowers and powders are flowers and powders whose use is permitted in the food sector. This embodiment is particularly adapted to provide a tracer for grain, which could not otherwise make contact with liquid or semiliquid tracers.

In a different embodiment, the tracer is applied to the product by applying a normal adhesive label, wherein the vector that contains the DNA is mixed in the adhesive composition that is present on the back of said label. In this embodiment, the application agent to be added to the tracer is selected among one or more adhesives that can be used for food.

In a different embodiment, the tracer, pure or mixed with one or more application agents, can be applied to the surface of the product after further liquid or gaseous dilution. Preferably, dilution occurs with substances that are acknowledged as being suitable for food use by applicable statutory provisions.

Although the list of substances cited above is not exhaustive, since they are all commonly used and easily commercially available, the person skilled in the art shall understand whether a substance that is not mentioned therein explicitly is in any case included therein implicitly by virtue of his normal general knowledge or by virtue of routine experiments. The person skilled in the art shall also be capable of selecting the application agent or agents that are most suited for the application technique to be performed by virtue of normal routine experiments.

The tracer according to the invention can be applied to the entire external surface of a certain product or only to a portion thereof. Advantageously, the surface to be used for marking and the extent of said surface to which application is to be made is chosen depending on the amount of tracer to be applied or on the type of industrial process to which the specific product will be subjected starting from its production up to its final use. In the embodiment in which the product to be marked is a food product, more particularly a horticultural product, one part that can be used advantageously to apply the tracer is one or more parts of the product that are not normally eaten. Parts that are not normally eaten are for example the stalk, the bones, thorns, leaves and stems.

The expression "commercial product" is used to designate any individual item or class of consumer goods (a set that is uniform or variable in terms of chemical and physical characteristics) for which it is desirable to be able to identify easily one or more of the characteristics mentioned above. Preferably, the product is a food product or a class of food products; more preferably, it is a horticultural product or a class of horticultural products. The fact that the tracer is to be applied to the surface of the product requires the product to be solid.

In another aspect, the invention relates to a method for identifying a commercial product as defined above.

The expression "user code" is used to designate an item or element that has a one-to-one match with the set of specific characteristics of the marked product that the user wishes to make available by means of tracer analysis. This set of information must be reported by the user to the administrator or creator of the database:
- when the user sends to the administrator the DNA from which the tracer is to be derived, or
- when a requesting user is assigned one or more tracers prepared by the database administrator or creator, or
- when a requesting user is assigned one or more tracer codes, each of which uniquely matches a single tracer prepared by the database administrator or creator.

The match between the user code and the information reported by the user is a one-to-one match, while the match between the tracer or tracer code and the user code is one-to-many, i.e., an individual user code can match two or more tracers or respective tracer codes, while a tracer or respective tracer code always and only matches a single user code (and therefore a single pool of information linked to said tracer code).

In a preferred embodiment, the step i) of providing one or more mutually different DNA traces comprises receiving one or more DNA fragments or a sample of DNA from which the one or more fragments to be inserted in the vector are to be derived. In this embodiment, the DNA is received from the subject who creates or administers the database (database administrator or creator), while the DNA is sent by the user. The received DNA can be derived partially or completely from the genetic code of the product to be identified or preferably from the genetic code of the user.

In a different embodiment, the step i) of providing one or more mutually different DNA tracers comprises generating the DNA from which the one or more fragments to be inserted in the vector are to be derived in a laboratory by synthetic or partly synthetic means (i.e., by coupling part of the DNA of the product or of the user with synthetic DNA). If the DNA is generated entirely by synthesis, during its production it is necessary to maintain procedures that are adapted to avoid the generation of two fragments that are identical both in terms of size and in terms of sequence. It will be immediately evident to the person skilled in the art which control methods are suitable for this purpose.

In a preferred embodiment, step ii) comprises a step iia) for storing said tracers in a database, a step iib) for creating, on the basis of each tracer stored in the database, an intermediate key or entry that corresponds uniquely to the tracer from which it originates (here also termed "tracer code"), and a step iic) for associating said key with a "user code" entry.

If the DNA tracer is completely or partially generated in the laboratory by the database creator or administrator, the "user code" entry is initially blank.

In a preferred embodiment, the method according to the invention comprises the steps of:
i) providing one or more mutually different DNA tracers as defined above,
   iia) storing said tracers in a database,
   iib) creating, on the basis of each tracer stored in the database, a tracer code that has a one-to-one match with the tracer from which it originates,
   iic) associating said tracer code with a "user code" entry,
iii) applying said tracer to the surface of a commercial product, preferably a food product, more preferably a horticultural product,
iv) recovering the tracer from the surface of said product,
v) analyzing the pattern of DNA inserts that are present in the recovered tracer,
vi) formulating a query based on the results of the analysis of the pattern of the DNA inserts in order to query the database so as to obtain the "user code" entry.

Since the user code, as mentioned above, is associated by a one-to-one match with a pool of information regarding the product supplied by said user, once the user code has been determined, one can also access the pool of information regarding the product that is associated therewith and is present in the database.

The query can be performed with any method suitable for the purpose, for example over the telephone or by means of computers.

The term "user" is used to designate both the manufacturer or manufacturers of a specific product as well as one or more of the vendors of the same product.

In another aspect, the invention relates to the use of a DNA tracer as defined above to provide surface identification of a commercial product to which it is applied.

All the aspects mentioned with reference to the tracer as such are to be considered valid, when applicable, also with reference to the method and the use according to the invention.

Other characteristics and advantages of the present invention will become better apparent from the description of the following preferred embodiments, intended exclusively by way of non-limiting example. Likewise, although only some preferred embodiments of the invention are described explicitly in the examples that follow as well as in the text, it will be immediately evident to the person skilled in the art that it is possible to modify some aspects described with reference to particular embodiments without thereby losing the advantages of the invention.

### Example 1

### Preliminary test for treating apples with plasmid DNA

By using splicing techniques that are standard in the biotechnology field, a sample was prepared of 250 µg of plasmid DNA modified by inserting DNA fragments not derived from the genetic makeup of the apples but derived from a gene bank available at the center where the tests on the invention were conducted (DIBIT research center of the San Raffaele hospital in Milan).

### Preliminary step

A solution A was prepared by diluting to 10 ml the DNA sample with water treated by distillation, UV and filtration.

The diluted plasmid DNA sample was then mixed with an appropriate quantity of wax constituted by a mixture of Carnauba wax and shellac in an aqueous emulsion, used typically in the food sector in order to coat fruit for protective purposes.

The mixture thus prepared was then applied to various groups of apples (hereinafter referenced with the letters B to L) by atomization. In particular, a fine atomizer (similar to the one commonly used to apply body fragrances) was used, holding the apple by its stalk, and the apples were wet uniformly with a specific number of sprays so as to apply to their surface a quantity of DNA-wax mixture that was always constant and approximately equal to 0.5 g of mixture per fruit.

### Group B

A solution B was prepared by drawing 6 ml of solution A as defined above (equal to approximately 150 µg of DNA) and by adding thereto approximately 19 ml of wax, so as to obtain a dosage of 6 µg (6000 ng) of DNA per ml of wax. Three apples were treated with the resulting solution B and one apple was treated with a blank (just wax without genetic material). Approximately 3000 ng of DNA were therefore applied to the surface of each apple.

### Group C

A solution C was prepared by drawing 5 ml of solution B and adding thereto approximately 45 ml of wax so as to obtain a dosage of 0.6 µg (600 ng) of DNA per ml of wax. Three apples were treated with the resulting solution C and one apple was treated with a blank. Approximately 300 ng of DNA were therefore applied to the surface of each apple.

### Group D

A solution D was prepared by drawing 5 ml of solution C and adding thereto approximately 45 ml of wax so as to obtain a dosage of approximately 0.06 µg (60 ng) of DNA per ml of wax. Three apples were treated with the resulting solution D, and one apple was treated with a blank. Approximately 30 ng of DNA were therefore applied to the surface of each apple.

### Group F

A solution E was prepared by drawing 5 ml of solution D and adding thereto approximately 45 ml of wax so as to obtain a dosage of approximately 0.006 µg (6 ng) of DNA per ml of wax. Three apples were treated with the resulting solution E, and one apple was treated with a blank. Therefore, approximately 3 ng of DNA were applied to the surface of each apple.

Two other groups of apples (groups K and L) were then treated with a wax constituted only by Carnauba wax without shellac, so as to verify the possible influence of the components of the waxy matrix on the integrity and subsequent identification of the tracer.

### Group K

A solution K was prepared by drawing 1 ml of solution A (equal to 25 µg of DNA) and by adding thereto approximately 49 ml of wax so as to obtain a dosage of approximately 0.5 µg (500 ng) of DNA per ml of wax. Three apples were treated with the resulting solution K, and one apple was treated with a blank. Approximately 250 ng of DNA were therefore applied to the surface of each apple.

### Group L

A solution L was prepared by drawing 5 ml of solution K and adding thereto approximately 45 ml of wax so as to obtain a dosage of approximately 0.05 µg (50 ng) of DNA per ml of wax. Three apples were treated with the resulting solution K and one apple was treated with a blank. Approximately 25 ng of DNA were therefore applied to the surface of each apple.

### Recovery and verification of the presence of the tracer

The DNA, as mentioned, can be recovered from the apples by means of methods, instruments and reagents that are conventional in the field of biotechnologies. The following method is described by way of example and performed for one of the apples of group C, on which there are approximately 300 theoretical ng of plasmid DNA.

Remove the wax with a scalpel from the peel of the apple. Collect the wax (approximately 50 mg) in a 1.5-ml Eppendorf tube that has been preweighed when empty. Add 500 µl of Tris 20 mM pH=9.5 and incubate at ambient temperature for 10 minutes. Add 500 µl of chloroform, invert five times and incubate under agitation at 50 °C for 15 minutes under a chemical hood. Centrifuge at 10,000 g for 10 minutes at ambient temperature. Collect the aqueous phase (upper clear phase) in a new 1.5-ml Eppendorf tube and add an identical volume of isopropanol at ambient temperature. Centrifuge at 10,000 g for 20 minutes at 4 °C. Remove the supernatant and wash the pellet with 75% ethanol at 4 °C. Centrifuge at 10,000 g for 20 minutes at 4 ° C. Remove the supernatant and dry the pellet. Resuspend the pellet in 10 µl of sterile water. Take 5 µl (containing approximately 37.5 ng of DNA) and perform the Polymerase Chain Reaction (PCR) method by using appropriate program and primers.

The result of the PCR was positive for the described apple as well as for the other described groups, confirming that the tracer can be prepared and applied with commonly used tools and ingredients and can be recovered and re-identified with methods that are absolutely standard in the field of recombinant DNA. Moreover, as demonstrated in the example described above, the products on which the tracer is applied can be treated in accordance with normal industrial procedures (for example, collection of the product, application of the wax with the tracer and direct sale) without these procedures requiring particular refinements or modifications for example in terms of ingredients. In particular, it has been demonstrated that the invention is a low-cost method that is simple to perform for any person skilled in the art.

## Claims

1. A DNA tracer, particularly for surface identification of a commercial product on which it is applied, said tracer comprising at least one type of DNA cloning vector, in which one or more fragments of DNA that are mutually different at least in terms of length or sequence are inserted, wherein:
- the inserted DNA is primary DNA,
- each inserted fragment has a length at least equal to 50 b.p.,
- if the inserted DNA derives entirely from the product to be identified, the inserts are at least two.

2. A method for identifying a commercial product, comprising the steps of:
i) providing one or more mutually different DNA tracers as defined above, and
ii) storing said tracers in a database, assigning uniquely to each tracer a "user code" entry.

3. The use of a DNA tracer as defined above to identify on the surface a commercial product on which the tracer is applied.

4. The invention according to any one of claims 1 to 3, wherein identifying a commercial product comprises detecting one or more of the following characteristics:
- general traceability of the product in the production chain,
- geographical production area,
- producer or vendor,
- production batch,
- characteristics of the processing to which the product has been subjected before its final use,
- state of preservation, if the product is of the food type,
- contamination with GMO (genetically modified organisms),
wherein if the detected characteristics are two or more, their detection occurs simultaneously.

5. The invention according to claim 4, wherein the product is a food product, preferably a horticultural product, and the detected characteristic is at least one among:
- general traceability of the product in the production chain,
- geographical production area,
- producer or vendor.

6. The invention according to any one of claims 1 to 3, wherein the vector is a plasmid.

7. The invention according to any one of claims 1 to 3, wherein the DNA fragments inserted in the vector are derived entirely from the genoma of the product to be identified and the fragments inserted in the vector are at least two.

8. The invention according to any one of claims 1 to 3, wherein the DNA fragments inserted in the vector have a length comprised between 50 b.p. and 1000 b.p., more preferably comprised between 100 b.p. and 1000 b.p.

9. The invention according to any one of claims 1 to 3, wherein the tracer comprises more than one vector type.

10. The invention according to any one of claims 1 to 3, wherein the DNA is selected among natural DNA, synthetic DNA, or a combination of both.

11. The invention according to any one of claims 1 to 3, wherein each fragment of DNA inserted in a vector is
- derived from the genetic code of the product to be identified, provided that said product contains DNA, or
- derived from the genetic makeup of the producer or of one of the producers, or
- derived from the genetic makeup of one or more of the vendors, or
- is created in the laboratory, on condition of not producing two fragments that are identical in terms of sequence and length, or
- the DNA inserts of a vector are at least two and are a combination of at least two of the following possibilities:
- DNA derived from the product,
- DNA derived from the producer, and
- totally or partially synthetic DNA.

12. The invention according to any one of claims 1 to 3, wherein the commercial product is a food product or a class of food products, more preferably a horticultural product.

13. The invention according to any one of claims 1 to 3, wherein the tracer also comprises at least one agent that is adapted to allow to apply the vector or vectors to the surface of the product to be identified.

14. The invention according to claim 13, wherein said agent is selected from the group that consists of:
- waxes and resins of vegetable and/or chemical origin and mixtures thereof normally used in the food sector,
- liquid and/or semiliquid substances of vegetable, animal, mineral and/or chemical origin normally used in the food sector,
- flours and powders, advantageously edible ones, preferably flours of vegetable, animal, chemical, mineral origin, and mixtures thereof,
- one or more adhesives that can be used for foods.

15. The method according to claim 2, wherein step i) comprises receiving one or more DNA fragments or a DNA sample from which the one or more fragments to be inserted in the vector are derived.

16. The method according to claim 2, wherein step i) comprises generating said one or more DNA fragments in the laboratory by synthetic or partially synthetic means, wherein if the DNA is completely generated by synthetic means it is necessary to maintain, during its production, procedures that are adapted to avoid the generation of two fragments that are identical in terms of both size and sequence.

17. The method according to claim 2, wherein step ii) comprises
iia) storing said one or more tracers in a database,
iib) creating, on the basis of each tracer stored in the database, an intermediate entry or key that corresponds uniquely to the tracer from which it has originated, and
iic) associating said key with a "user code" entry.

18. The method according to claim 2, comprising the steps of:
i) supplying one or more mutually different DNA traces, defined as in claim 1,
iia) storing said one or more tracers in a database,
iib) creating, on the basis of each tracer stored in the database, an intermediate entry or key, referred to as tracer code having a on-to-one match with the tracer from which it originated,
iic) associating said tracer code with an entry known as user code,
iii) applying tracer defined as in claim 1 to the surface of a commercial product, preferably a food product, more preferably a horticultural product,
iv) recovering the tracer from the surface of said product,
v) analyzing the pattern of DNA inserts present in the recovered tracer,
vi) formulating a query that is based on results of the analysis of the pattern of the DNA inserts in order to query the database so as to obtain the "user code" entry.

19. The method according to claim 18, wherein the tracer is applied according to one of the following possibilities:
- pure, or
- mixed with an agent as defined in claim 14.

20. The method according to claim 19, wherein the tracer is applied after further dilution, performed:
- in a liquid, or
- in a gas.

21. The method according to claim 18, wherein the tracer is applied to the entire or partial surface of the commercial product to be identified.

22. The method according to claim 18, wherein the product is a food product, preferably a horticultural product, and the tracer is applied to one or more parts that are not normally eaten.

23. The method according to claim 18, wherein the tracer is applied to a product according to one or more of the following methods:
- full or partial immersion of the product,
- full or partial atomization of the surface of the product,
- full or partial spreading of the surface of the product.

## Amended claims

### Amended claims in accordance with Rule 86(2) EPC.

**1.** A DNA tracer, particularly for surface identification of a commercial product on which it is applied, said tracer comprising at least one type of DNA cloning vector, in which one or more fragments of DNA that are mutually different at least in terms of length or sequence are inserted, wherein:
- the inserted DNA is primary DNA,
- each inserted fragment has a length at least equal to 50 b.p., and
- each fragment of DNA inserted in a vector is:
- derived from the genetic makeup of the producer or of one of the producers, or
- derived from the genetic makeup of one or more of the vendors, or
- the DNA inserts of a vector are at last two and are a combination of at least two of the following possibilities:
- DNA derived from the product,
- DNA derived from the producer, and
- totally or partially synthetic DNA,
where DNA derived from the producer must be present.

**2.** A method for surface identification of a commercial product, comprising the steps of:
i) supplying one or more mutually different DNA tracers, wherein the DNA tracer comprises at least one type of DNA cloning vector, in which one or more fragments of DNA that are mutually different at least in terms of length or sequence are inserted, and wherein:
- the inserted DNA is primary DNA,
- each inserted fragment has a length at least equal to 50 b.p.,
- if the inserted DNA derives entirely from the product to be identified, the inserts are at least two,
iia) storing said one or more tracers in a database,
iib) creating, on the basis of each tracer stored in the database, an intermediate entry or key, referred to as tracer code having a one-to-one match with the tracer from which it originated,
iic) associating said tracer code with an entry known as user code,
iii) applying tracer defined as in claim 1 to the surface of a commercial product, preferably a food product, more preferably a horticultural product,
iv) recovering the tracer from the surface of said product,
v) analyzing the pattern of DNA inserts present in the recovered tracer,
vi) formulating a query that is based on results of the analysis of the pattern of the DNA inserts in order to query the database so as to obtain the "user code" entry.

**3.** The use of a DNA tracer as defined in claim 1 for surface identification of a commercial product on which the tracer is applied.

**4.** The invention according to any one of claims 1 to 3, wherein surface identification of a commercial product comprises the detection one or more of the following characteristics:
- general traceability of the product in the production chain,
- geographical production area,
- producer or vendor,
- production batch,
- characteristics of the processing to which the product has been subjected before its final use,
- state of preservation, if the product is of the food type,
- contamination with GMO (genetically modified organisms),
wherein if the detected characteristics are two or more, their detection occurs simultaneously.

**5.** The invention according to claim 4, wherein the product is a food product, preferably a horticultural product, and the detected characteristic is at least one among:
- general traceability of the product in the production chain,
- geographical production area,
- producer or vendor.

**6.** The invention according to any one of claims 1 to 3, wherein the vector is a plasmid.

**7.** The invention according to any one of claims 1 to 3, wherein the DNA fragments inserted in the vector have a length comprised between 50 b.p. and 1000 b.p., more preferably comprised between 100 b.p. and 1000 b.p.

**8.** The invention according to any one of claims 1 to 3, wherein the tracer comprises more than one vector type.

**9.** The invention according to any one of claims 1 to 3, wherein the commercial product is a food product or a class of food products, more preferably a horticultural product.

**10.** The invention according to any one of claims 1 to 3, wherein the tracer also comprises at least one agent that is adapted to allow to apply the vector or vectors to the surface of the product to be identified.

**11.** The invention according to claim 10, wherein said agent is selected from the group that consists of:
- waxes and resins of vegetable and/or chemical origin and mixtures thereof normally used in the food sector,
- liquid and/or semiliquid substances of vegetable, animal, mineral and/or chemical origin normally used in the food sector,
- flours and powders, advantageously edible ones, preferably flours of vegetable, animal, chemical, mineral origin, and mixtures thereof,
- one or more adhesives that can be used for foods.

**12.** The method according to claim 2, wherein the DNA fragments inserted in the vector are derived entirely from the genoma of the product to be identified and the fragments inserted in the vector are at least two.

**13.** The method according to claim 2, wherein the DNA is selected among natural DNA, synthetic DNA, or a combination of both.

**14.** The method according to claim 2, wherein each fragment of DNA inserted in a vector is
- derived from the genetic code of the product to be identified, provided that said product contains DNA, or
- derived from the genetic makeup of the producer or of one of the producers, or
- derived from the genetic makeup of one or more of the vendors, or
- is created in the laboratory, on condition of not producing two fragments that are identical in terms of sequence and length, or
- the DNA inserts of a vector are at least two and are a combination of at least two of the following possibilities:
- DNA derived from the product,
- DNA derived from the producer, and
- totally or partially synthetic DNA.

**15.** The method according to claim 2, wherein step i) comprises receiving one or more DNA fragments or a DNA sample from which the one or more fragments to be inserted in the vector are derived.

**16.** The method according to claim 2, wherein step i) comprises generating said one or more DNA fragments in the laboratory by synthetic or partially synthetic means, wherein if the DNA is completely generated by synthetic means it is necessary to maintain, during its production, procedures that are adapted to avoid the generation of two fragments that are identical in terms of both size and sequence.

**17.** The method according to claim 2, wherein the tracer is applied according to one of the following possibilities:
- pure, or
- mixed with an agent as defined in claim 14.

**18.** The method according to claim 17, wherein the tracer is applied after further dilution, performed:
- in a liquid, or
- in a gas.

**19.** The method according to claim 2, wherein the tracer is applied to the entire or partial surface of the commercial product to be identified.

**20.** The method according to claim 2, wherein the product is a food product, preferably a horticultural product, and the tracer is applied to one or more parts that are not normally eaten.

**21.** The method according to claim 2, wherein the tracer is applied to a product according to one or more of the following methods:
- full or partial immersion of the product,
- full or partial atomization of the surface of the product,
- full or partial spreading of the surface of the product.
